(19) Europäisches Patentamt
European Patent Office
Office européen des brevets

(11) Veröffentlichungsnummer: **0 031 426**
**B1**

(12) # EUROPÄISCHE PATENTSCHRIFT

(45) Veröffentlichungstag der Patentschrift: **15.02.84**

(51) Int. Cl.³: **C 07 D 307/935**, A 61 K 31/34

(21) Anmeldenummer: **80106884.2**

(22) Anmeldetag: **08.11.80**

(54) **7-Oxo-PGI2-derivate, Verfahren zu deren Herstellung sowie diese Verbindungen enthaltende pharmazeutische Präparate.**

(30) Priorität: **12.11.79 HU CI001987**
**12.11.79 HU CI001988**

(43) Veröffentlichungstag der Anmeldung: **08.07.81 Patentblatt 81/27**

(45) Bekanntmachung des Hinweises auf die Patenterteilung: **15.02.84 Patentblatt 84/7**

(84) Benannte Vertragsstaaten: **BE CH DE FR GB IT LI NL SE**

(56) Entgegenhaltungen:
**TETRAHEDRON LETTERS, Nr. 9, Februar 1979, Seiten 805-808, Pergamon Press, Oxford, New York, Paris, Frankfurt H. NAKAI et al.: "Synthesis of nitrogen-containing prostaglandin 11 analogs"**

(73) Patentinhaber: **CHINOIN Gyogyszer és Vegyészeti Termékek Gyára RT., To utca 1-5, H-1045 Budapest V (HU)**

(72) Erfinder: **Simonidesz, Vilmos, Dr. Dipl.Ing.Chem.,, H-1016 Fenyö u. 13, Budapest (HU)**
Erfinder: **Papp geb. Behr, Agnes, Dipl.Ing.Chem., H-1041 Deák F. u. 33, Budapest (HU)**
Erfinder: **Kovács, Gábor, Dr. Dipl.Ing.Chem., H-1142 Róna park 4, Budapest (HU)**
Erfinder: **Ivanics, József, Dipl.Ing.Chem,, H-1045 Elem u.20, Budapest (HU)**
Erfinder: **Dér geb. Földváry, Julia, Dipl.Ing.Chem., H-1093 Szamuely u. 41, Budapest (HU)**
Erfinder: **Stadler, István, Dr., H-1143 Népstadion u. 18, Budapest (HU)**
Erfinder: **Pallagi, István, Ady E. u. 105, H-1188 Budapest (HU)**

(74) Vertreter: **Lotterhos, Hans Walter, Dr.-Ing., Lichtensteinstrasse 3, D-6000 Frankfurt am Main 1 (DE)**

EP 0 031 426 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 7-Oxo-$PGI_2$-derivate, Verfahren zu deren Herstellung sowie diese Verbindungen enthaltende pharmazeutische Präparate

Die Erfindung betrifft neue optisch aktive oder racemische 7-Oxo-$PGI_2$-derivate der allgemeinen Formel

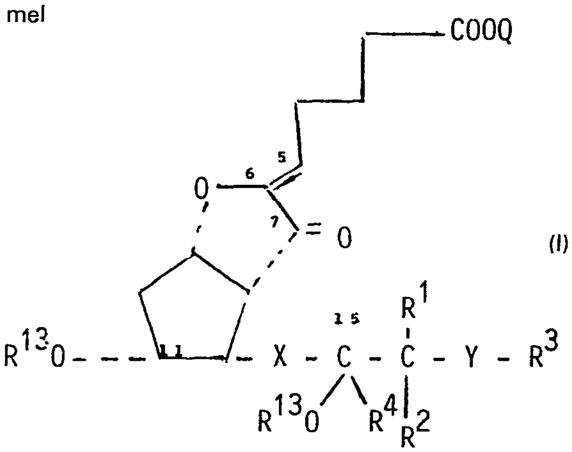

(I)

worin

Q Wasserstoff, ein physiologisch verträgliches Kation oder $C_{1-6}$-Alkyl,

X cis- oder trans-CH=CH, -C≡C- oder $-CH_2-CH_2-$,

$R^{13}$ Wasserstoff, $C_{1-4}$-Alkanoyl, eine Schutzgruppe der allgemeinen Formel
$R^7R^8R^9Si$- oder $R^{11}CH_2$-C($R^{10}$)- bedeuten,
$OR^{12}$

worin $R^7$, $R^8$ und $R^9$, die gleich oder verschieden sein können, $C_{1-4}$-Alkyl,

$R^{10}$ und $R^{11}$, die gleich oder verschieden sein können, Wasserstoff oder Methyl und

$R^{12}$ Methyl oder Äthyl bedeuten oder die Tetrahydro-pyran-2-yl-gruppe,

$R^4$ Wasserstoff, $C_{1-6}$-Alkyl in sterischer $\alpha$- oder $\beta$-Konfiguration,

$R^1$ und $R^2$ Wasserstoff oder $C_{1-6}$-Alkyl,

Y Methylen, Sauerstoff oder -NH- und

$R^3$ $C_{1-6}$-Alkyl oder Phenyl, das durch Halogen substituiertes $C_{1-6}$-Alkyl, Hydroxyl, Sulfhydryl, $C_{1-6}$-Alkanoyloxy, Benzoyloxy, Amino, Mono- oder Di-$C_{1-6}$-alkylamino, Halogen, $C_{1-6}$-Alkoxy oder $C_{1-6}$-Alkylthio monosubstituiert sein kann, bedeuten,

sowie Verfahren zur Herstellung dieser Verbindungen und diese Verbindungen enthaltende pharmazeutische Präparate.

Die neuen Verbindungen der allgemeinen Formel I sind biologisch aktive Verbindungen, die als stabilisierte Analoga des ausserordentlich wirksamen natürlichen $PGI_2$ angesehen werden können. $PGI_2$ (Prostacyclin) wurde erstmalig in Nature, *263*, 663 (1976) beschrieben. Es stellt eines der wichtigsten Kettenglieder des Arachidonsäure-Metabolismus dar und ist schon in sehr geringer Menge wirksam. Unter den bemerkenswerten pharmakologischen Wirkungen ist insbesondere die aggregationshemmende und blutdrucksenkende Wirkung zu erwähnen. $PGI_2$ verhindert schon in Mengen von Nanogrammen die Aggregation der Blutplättchen und weist eine thrombolytische Wirkung bei dem aggregierten Thrombus auf. Infolge dieser speziellen pharmakologischen Wirkung stellt $PGI_2$ ein sehr wertvolles Pharmakon bei der Behandlung der Krankheit des Jahrhunderts: der Thrombose und der abnormalen Hämostase dar. Die klinische Anwendung dieser Verbindung ist schon in einer grossen Anzahl von Artikeln beschrieben worden.

Das grösse Problem bei der Anwendung von $PGI_2$ als Arzneimittel stellt seine ausserordentlich starke Instabilität dar. In saurem oder neutralem Medium zersetzt sich $PGI_2$ unter Bildung von 6-Keto-$PGF_{1\alpha}$ im Gleichgewicht mit der Hemiketal-Form. $PGI_2$ enthält eine sehr reaktionsfähige Enolàthergruppierung, die durch das O-Atom des Furanrings und die 5,6-Doppelbindung der Seitenkette gebildet wird und die mit der äusseren oder inneren Protonenquelle gleich stark reagiert. $PGI_2$ ist infolgedessen in Form der freien Säure nicht beständig, es wird vielmehr in Form seiner Salze und Ester bei den pharmakologischen und klinischen Untersuchungen verwendet.

Die sehr reaktionsfähige Enolätherkonfiguration ist aber nicht nur Ursache für die starke chemische Instabilität des natürlichen $PGI_2$, sie ist auch Träger der starken Aggregationshemmwirkung des $PGI_2$ auf die Blutplättchen. Infolgedessen wurde bei der Entwicklung von neuen $PGI_2$-derivaten, die eine für den praktischen Gebrauch ausreichende Stabilität aufweisen, gewöhnlich so vorgegangen, dass die labile Enolätherkonfiguration durch eine andere chemisch stabile Konfiguration ersetzt und durch Ausbalancieren der beiden Faktoren: Wirkungsverlust gegenüber erzielter Molekülstabilität, versucht wurde, ein Optimum zu erzielen.

So beruht bei den aus Tetrahedron Lett. Nr. 9, S. 805/808 (1979) bekannten 6-Aza-6,9-methano-$PGI_1$-derivaten, die gegenüber dem $PGI_2$ wesentlich verbesserte Stabilität darauf, dass der Furanring des $PGI_2$ durch einen Pyrrolidinring ersetzt und die Seitenkette durch eine Einfachbindung mit dem Ringstickstoff verbunden worden ist. Gegebenenfalls in den Pyrrolidinring eingeführte Oxogruppen führten nicht zu einer weiteren Erhöhung der Molekülstabilität. Die Aggregationshemmwirkung der so erhaltenen Verbindungen ist jedoch nur schwach.

Ziel der Erfindung war, nicht mehr instabile $PGI_2$-derivate herzustellen, d.h. solche Derivate, die eine für den praktischen Gebrauch als Pharmazeuticum ausreichende Stabilität aufweisen, ohne die für die Aggregationshemmwirkung wichtige Enolätherkonfiguration zu verändern.

Dieses Ziel ist gemäss Erfindung mit der Einführung der Oxogruppe in die 7-Stellung des $PGI_2$-Moleküls erreicht worden. Die neuen, optisch aktiven oder racemischen 7-Oxo-$PGI_2$-derivate der allgemeinen Formel I weisen bei wesentlich verbesserter Stabilität die vorteilhaften pharmazeutischen Eigenschaften des $PGI_2$, wie die Thrombozytenaggregation hemmende und die thrombolytische Aktivität auf.

In der allgemeinen Formel I ist unter physiologisch verträglichem Kation (eine Bedeutung von Q) jedes organische oder anorganische Kation zu verstehen, das in den angewendeten Dosen keine schädigende

(toxische) Wirkung ausübt. Einige Beispiele für solche anorganische Kationen sind: Alkaliionen, z.B. Natrium-, Kalium- oder Ammoniumionen, oder Erdalkalimetallionen, z.B. Calcium- oder Magnesiumionen. Einige Beispiele für organische Kationen sind: durch organische Gruppen, vorzugsweise Alkylgruppen, substituierte Ammoniumionen, wobei die Substituenten der Ammoniumionen zur günstigen Beeinflussung des Lösungsvermögens und Kristallisierungsvermögens der Salze weitere Substituenten, z.B. Hydroxygruppen oder Aminogruppen, tragen können. Ein Beispiel für ein solches Kation ist das Tris-(hydroxymethyl)-ammoniumion.

Unter den Alkylgruppen mit 1-6 Kohlenstoffatomen sind geradkettige oder verzweigte Gruppen zu verstehen. Einige Beispiele hierfür sind: Methyl, Äthyl und iso-Propyl, n-, sec.-, tert.- und iso-Butyl, die verschiedenen Pentyl- und Hexylgruppen. Die bevorzugten Alkylgruppen enthalten 1-4 Kohlenstoffatome.

Unter den Hydroxyl-Schutzgruppen $R^6$ sind die in der Prostaglandinchemie bekannten Schutzgruppen zu verstehen, deren Einführung in die Hydroxylgruppe und deren Entfernung mittels der aus der Literatur bekannten Methoden erfolgen können. Die Einführung der Schutzgruppen ist zweckmässig, damit bei der Oxydation der Verbindungen der allgemeinen Formel

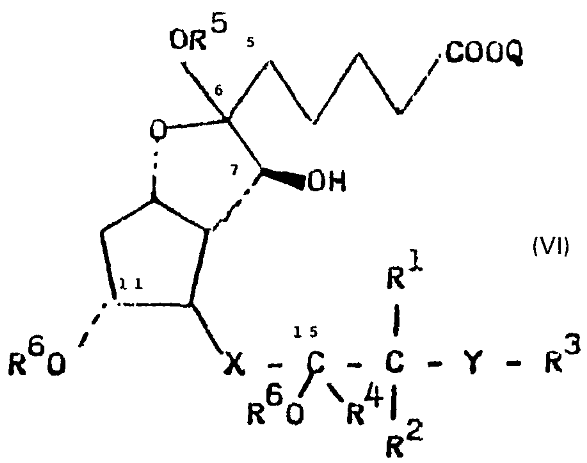

(VI)

nur die Hydroxylgruppe in der 7-Stellung oxydiert wird.

Die Schutzgruppen können nach der Oxydation sowie nach der Abspaltung des Alkohols $R^5OH$ entfernt werden.

Monosubstituiertes Phenyl — änstelle von $R^3$ — kann eine oder mehrere der nachfolgend aufgezählten Substituenten — die bei Anwesenheit mehrerer Substituenten gleich oder verschieden sein können — enthalten: gegebenenfalls durch ein oder mehrere Halogenatome substituiertes $C_{1-6}$-Alkyl, Hydroxyl, Sulfhydryl, $C_{1-6}$-Alkanoyloxy, Benzoyloxy, Amino, Mono- oder Di-$C_{1-6}$-alkylamino, Halogen, $C_{1-6}$-Alkoxy oder $C_{1-6}$-Akylthio.

Die Verbindungen der allgemeinen Formel I können gemäss Erfindung z.B. aus den Verbindungen der allgemeinen Formel

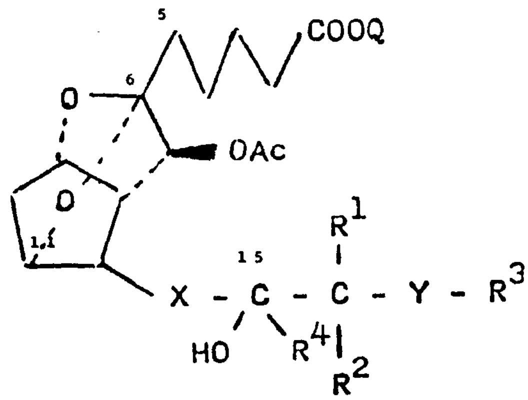

(III)

worin $R^1$, $R^2$, $R^3$, X, Y und Q die oben angegebene Bedeutung haben, und

Ac Acetyl ist,

hergestellt werden. Die Verbindungen der allgemeinen Formel III sind bekannt [J. Am. Chem. Soc., *100*, 6756 (1978)] oder können aus den entsprechenden Ausgangsverbindungen durch die aus dieser Veröffentlichung bekannten Methoden hergestellt werden.

Die Verbindungen der allgemeinen Formel III werden gemäss Erfindung mit einem niederen Alkohol, vorzugsweise Methanol, in Gegenwart einer katalytischen Menge eines Säurekatalysators bei −78°C bis +25°C umgesetzt. Als Säurekatalysator können Mineralsäuren, Sulfonsäuren oder Lewis-Säuren eingesetzt werden; bevorzugt wird Bortrifluoridätherat.

Die isomeren 6-Ketale der allgemeinen Formel

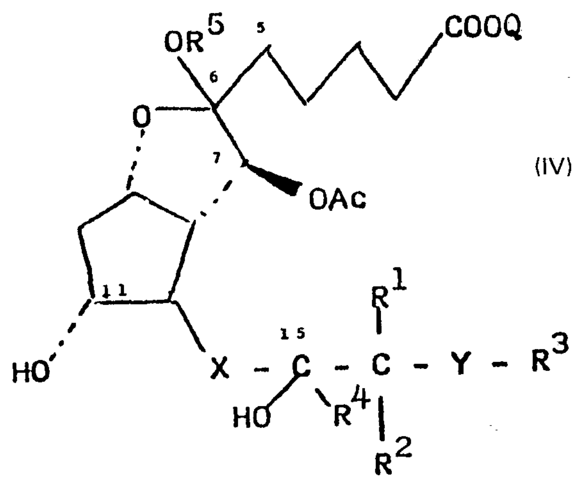

(IV)

worin $R^1$, $R^2$, $R^3$, $R^4$, X, Y, Q und Ac die oben angegebene Bedeutung haben und

$R^5$ $C_{1-6}$-Alkyl ist,

können in Form ihrer Gemische oder nach chromatographischer Trennung in der nachfolgenden Reaktion eingesetzt werden.

Um die 7-Acetoxygruppe selektiv zu desacetylisieren und die gebildete Hydroxylgruppe selektiv zu oxydieren werden die freien 11- und 15-Hydroxylgruppen der Verbindungen der allgemeinen Formel IV vorübergehend durch beständige Schutzgruppen geschützt, wobei die Verbindungen der allgemeinen Formel IV in alkalischem oder basischem Medium, vorzugsweise mit

a) einem Trialkylchlorsilan der allgemeinen Formel

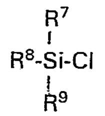

(VII)

worin $R^7$, $R^8$ und $R^9$ gleiche oder verschiedene geradkettige oder verzweigte $C_{1-4}$-Alkylgruppen bedeuten,
oder mit
b) einem Enol-äther der allgemeinen Formel

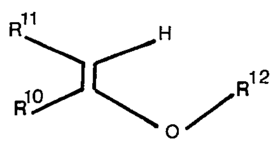

(VIII)

worin $R^{10}$ und $R^{11}$, die gleich oder verschieden sein können, und Wasserstoff oder Methyl und
$R^{12}$ Methyl oder Äthyl bedeuten,
oder
c) mit 3,4-Dihydro-2H-pyran umgesetzt werden.

Als Silylierungsmittel der allgemeinen Formel VII kann man Trialkylchlorsilane, z.B. Dimethyl-tert.--butyl-chlorsilan, Trimethylchlorsilan, verwenden, wobei das Dimethyl-tert.-butyl-chlorsilan in aprotischen Lösungsmitteln, vorzugsweise in Dimethylformamid, in Gegenwart von Katalysatoren, vorzugsweise Imidazol, eingesetzt und das Trimethylchlorsilan in Pyridin oder in aprotischen Lösungsmitteln, vorzugsweise in Gegenwart von tertiären Aminen, verwendet wird. Die Reaktionstemperatur kann in einem Bereich zwischen −20 und +60°C variiert werden.

Als Enol-äther der allgemeinen Formel VIII werden vorzugsweise Äthyl-vinyl-äther, iso-Propenyl-methyl-äther in aprotischen Lösungsmitteln, insbesondere in Dichlormethan, in Gegenwart einer katalytischen Menge einer Säure verwendet. Als Katalysatoren werden Mineralsäuren, Phosphorylchlorid, Sulfonsäuren, vorzugsweise p-Toluol-sulfonsäure, eingesetzt. Die Reaktionstemperatur kann in einem Bereich zwischen −78 bis +50°C variiert werden.

Anstelle der Enol-äther der allgemeinen Formel VIII kann man auch das 3,4-Dihydro-2H-pyran verwenden.

Die so erhaltenen, geschützte Hydroxylgruppen aufweisenden Verbindungen der allgemeinen Formel

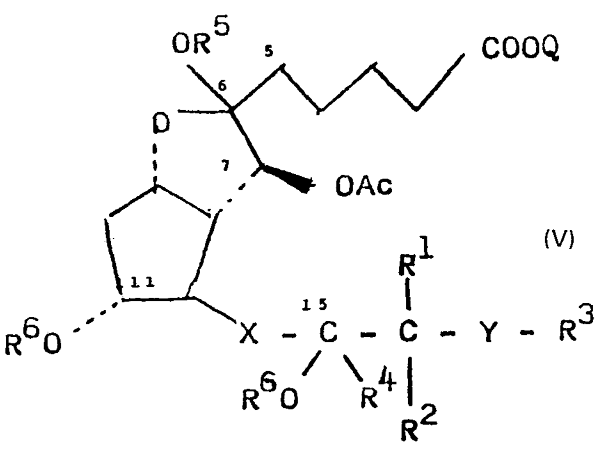

(V)

worin $R^1$, $R^2$, $R^3$, $R^4$, $R^5$, X, Y, Ac und Q die oben angegebene Bedeutung haben und
$R^6$ eine Silylgruppe der Formel $R^7R^8R^9Si$-, eine α-Alkoxyalkylgruppe der Formel $R^{11}CH_2$-C($R^{10}$)- oder eine Tetrahydropyran-2-yl-gruppe $OR^{12}$ darstellt und
worin $R^7$, $R^8$, $R^9$, $R^{10}$, $R^{11}$ und $R^{12}$ die oben angegebene Bedeutung haben,
können in einem basischen Medium zu Verbindungen der allgemeinen Formel VI hydrolysiert werden, wobei die 7-Acetylgruppe in niederen Alkanolen, vorzugsweise Methanol, in Gegenwart von milden Basen, vorzugsweise Kaliumcarbonat, oder mit wässrigen Lösungen von Alkali- oder Erdalkalihydroxyden hydrolytisch abgespalten wird. Im letztgenannten Fall werden die aus dem Reaktionsgemisch isolierten Carbonsäuren wieder in ihre niederen Alkylester, vorzugsweise — durch Verwendung von Diazomethan — in ihre Methylester, übergeführt. Die Reaktionstemperatur kann zwischen 0°C und dem Siedepunkt des eingesetzten Lösungsmittels variiert werden, vorzugsweise arbeitet man bei Raumtemperatur. Bei längeren Reaktionszeiten und in einem stark basischen Reaktionsmedium kann die Alkylgruppe $R^5$ durch Wasserstoff ersetzt werden.

Die Oxydation der Hydroxylgruppe in 7-Stellung der Verbindungen der allgemeinen Formel

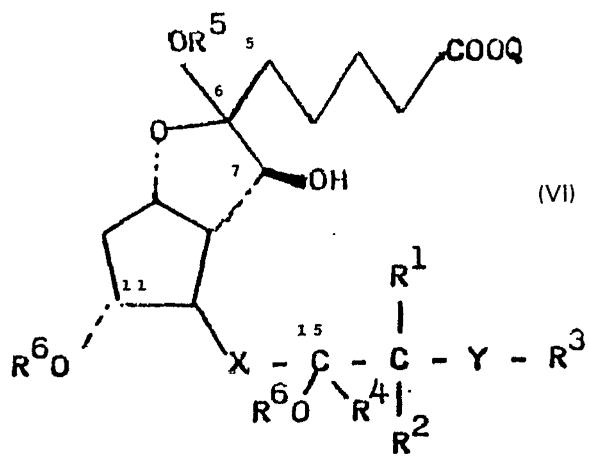

(VI)

worin $R^5$ durch Wasserstoff ersetzt ist oder $C_{1-6}$-Alkyl bedeutet,
kann mit jedem Oxydationsmittel — das eine sekundäre Hydroxylgruppe zur Oxogruppe zu oxydieren vermag, z.B. Jones-Reagens, Collins-Reagens, Thioanisol-Chlor-Komplex, Pyridiniumchlorchromat usw. — wobei dem Pyrimidiniumchlorchromat der Vorzug gegeben wird — in aprotischen Lösungsmitteln, vorzugsweise in chlorierten Kohlenwasserstoffen, durchgeführt werden. Die Reaktionstemperatur kann zwischen −40 und +50°C variiert werden.

Werden Verbindungen der allgemeinen Formel VI oxydiert, worin $R^5$ durch Wasserstoff ersetzt ist, findet eine selektive Oxydation der Hydroxylgruppe in 7-Stellung statt, wobei man Hemiketal-derivate der allgemeinen Formel

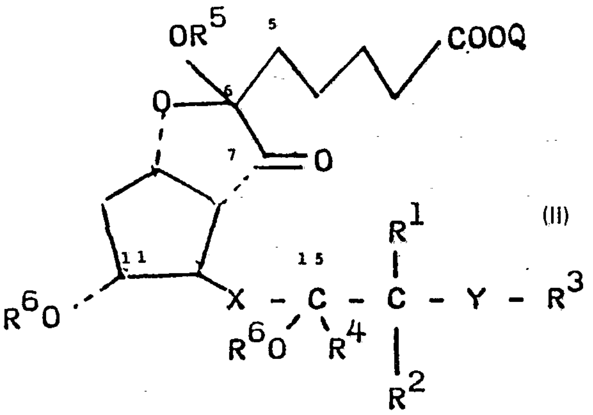

(II)

erhält, in denen $R^5$ durch Wasserstoff ersetzt ist. Diese Verbindungen können weiter umgesetzt oder — gewünschtenfalls — in Verbindungen der allgemeinen Formel II übergeführt werden, in denen $R^5$ $C_{1-6}$-Alkyl bedeutet.

In den durch Oxydation erhaltenen Verbindungen der allgemeinen Formel II können gewünschtenfalls die Schutzgruppen $R^6$ abgespalten und die freien 11- und 15-Hydroxylgruppen mit einer $C_{1-4}$-Alkanoylgruppe acyliert werden.

In der Reaktionsstufe — der Ausbildung der 5,6-Doppelbindung — können sowohl durch die $R^6$-Gruppe geschützte, als auch acylierte und freie 11- und 15-Hydroxylgruppen enthaltende Verbindungen der allgemeinen Formel II eingesetzt werden.

Die 7-Oxo-$PGI_2$-derivate der allgemeinen Formel I werden aus den 7-Oxo-derivaten der allgemeinen Formel II durch Abspalten des Alkohols der allgemeinen Formel $R^5$-OH erhalten.

Dieser Alkoholabspaltung können auch Verbindungen der allgemeinen Formel II, worin $R^5$ $C_{1-6}$-Alkyl bedeutet und $R^6$ durch Wasserstoff ersetzt ist, unterworfen werden, wobei Verbindungen der allgemeinen Formel I, worin $R^{13}$ Wasserstoff ist, erhalten werden. Die Alkoholabspaltung kann durch Erhitzen in verschiedenen dipolaren aprotischen Lösungsmitteln, z.B. Dimethylformamid, Dimethylsulfoxyd, Hexamethyl-phosphorsäure-triamid — wobei dem Hexamethyl-phosphorsäure-triamid der Vorzug gegeben wird — vorgenommen werden. Die Reaktionstemperatur kann im Bereich zwischen 70 und 180°C, vozugsweise zwischen 80 und 160°C, variiert werden. Die Alkoholabspaltung kann durch Verwendung von Alkansäureanhydriden mit 1-4 Kohlenstoffatomen im Alkylteil, Benzol oder Toluol beschleunigt werden. Bei Verwendung von Säureanhydriden entstehen auch Verbindungen der allgemeinen Formel I, worin $R^{13}$ Acyl bedeutet, das gewünschtenfalls durch eine durch Basen katalysierte Alkoholyse entfernt werden kann.

Verbindungen der allgemeinen Formel I, in denen Q = $C_{1-6}$-Alkyl bedeutet und die nach der Alkoholabspaltung erhalten worden sind, können z.B. nach säulenchromatographischer Reinigung isoliert werden.

Verbindungen der allgemeinen Formel I, in denen anstelle von $R^6$ $R^{13}$ steht, können auch aus Verbindungen der allgemeinen Formel II, in denen $R^6$ durch Wasserstoff ersetzt ist und $R^5$ $C_{1-6}$-Alkyl bedeutet, durch die oben angegebene Alkoholabspaltung erhalten werden.

$R^6$ kann für die weiter oben angegebenen Hydroxylschutzgruppen die Bedeutung von z.B. Trialkylsilyl-, Alkoxy-alkyl haben. Die Verbindungen der allgemeinen Formel I, in denen $R^{13}$ eine $R^6$-Schutzgruppe bedeutet, können nach säulenchromatographischer Reinigung isoliert und gewünschtenfalls nach Entfernung der Schutzgruppen in Verbindungen der allgemeinen Formel I übergeführt werden, in denen $R^{13}$ Wasserstoff bedeutet. Trialkyl-silyl-Schutzgruppen werden bevorzugt mit Tetrabutylammoniumfluorid in Lösungsmitteln vom Äther-Typ, vorzugsweise Tetrahydrofuran, bei einer Temperatur von −20 bis +50°C, vorzugsweise bei Raumtemperatur, entfernt.

Aus den Verbindungen der allgemeinen Formel II, die anstelle von $R^5$ Wasserstoff enthalten und in denen $R^6$ die oben angegebene Schutzgruppen bedeutet, können die 7-Oxo-$PGI_2$-derivate der allgemeinen Formel I durch Abspalten von Wasser, das zweckmässig durch azeotrope Destillation entfernt wird, erhalten werden. Die Wasserabspaltung durch azeotrope Destillation wird zweckmässig durch Kochen in aromatischen Kohlenwasserstoffen, vorzugsweise Benzol, in Gegenwart von wasserbindenden Mitteln, vorzugsweise wasserfreiem Magnesiumsulfat durchgeführt. Nach Entfernung der Schutzgruppen $R^6$ werden die Verbindungen der allgemeinen Formel I, worin $R^{13}$ Wasserstoff und Q niederes Alkyl bedeuten, z.B. durch Chromatographieren gereinigt.

Verbindungen der allgemeinen Formel I, in denen Q ein physiologisch verträgliches Kation bedeutet, werden aus den entsprechenden Estern der allgemeinen Formel I, in denen Q = $C_{1-6}$-Alkyl ist, durch wässrige alkalische Hydrolyse erhalten. Die Hydrolyse wird zweckmässig mit wässrigen Alkali- und Erdalkalimetallhydroxydlösungen bei 0 bis 100°C durchgeführt. Die Salze werden z.B. nach dem Lyophilisieren isoliert. Aus den wässrigen Lösungen der Salze können die Carbonsäuren durch vorsichtiges Ansäuern freigesetzt, in organischen Lösungsmitteln und mit aliphatische und aromatischen Aminen in weitere Salze übergeführt werden.

Die erfindungsgemässen Verbindungen der allgemeinen Formel I hemmen die Aggregation der Thrombocyten und weisen eine thrombolytische Wirkung auf, wie das natürliche $PGI_2$. Die neuen Verbindungen hemmen die Synthese des Thromboxan $A_2$, die Wirkung des Thromboxan $A_2$, die Magensäuresekretion und beeinflussen einige Kreislaufprozesse. Die Verbindungen können zur Heilung von Asthma, von myokardialem Infarkt, Thrombose, Magengeschwäuren und vaskulären Unregelmässigkeiten bei Säugetieren, insbesondere bei Menschen, verwendet werden.

Pharmazeutische Präparate der Erfindung, die als Wirkstoff 7-Oxo-$PGI_2$-derivate enthalten, haben eine thrombolytische Wirkung und können gleichzeitig die Gefahr des Entstehens der Thrombi ausschalten. Dies ist von besonders grosser Bedeutung bei der Blutkonservierung, bei der Perfusion von Organen, Geweben und bei der Ausschaltung der Thrombose-Gefahr nach Operationen.

Mit einem an Thrombocyten angereicherten, aus

Humanblut isolierten Plasma (PRP) wurden bei der durch ADP oder Kollagen verursachten Aggregation die folgenden $ID_{50}$-Konzentrationen erhalten:

| Aggregations-erzeugende Verbindung | Hemmwirkung auf die Thrombocyten-aggregation $ID_{50}$ (ng/ml) | | |
|---|---|---|---|
| | $PGI_2$-Natriumsalz | 7-Oxo-$PGI_2$-Natriumsalz | 7-Oxo-$PGI_2$-methylester |
| ADP | 1 -2 | 15 | 100 - 200 |
| Kollagen | 1 - 2 | 80 | 50 |

Es ist sehr wichtig, dass die Kontraktionswirkung des 7-Oxo-$PGI_2$-derivats der Erfindung auf den Magenfundus 0,1-mal grösser als die des $PGI_2$ ist. In der Hemmwirkung auf die Thrombocytenaggregation sind die 7-Oxo-$PGI_2$-derivate der Erfindung — wie die Angaben weiter oben zeigen — dem natürlichen $PGI_2$ nur geringfügig unterlegen, in der Stabilität dagegen wesentlich überlegen, was die Verwendung der 7-Oxo-$PGI_2$-derivate in pharmazeutischen Zubereitungen ermöglicht.

Die Dosis der 7-Oxo-$PGI_2$-derivate der allgemeinen Formel I pro Tag beträgt 100 ng bis 100 mg, und hängt von der Höhe der zu erreichenden Wirkung sowie von der Applikationsart, der Empfindlichkeit der behandelten Person, des Tieres, der Organe und der Gewebe ab. Bei kontinuierlicher Infusion ist im allgemeinen eine Dosis von 1 bis 10 mg pro Tag genügend.

Die Präparate können in flüssiger, halb-flüssiger Form, in Form von Salben, Pasten vorliegen.

Als flüssige Zubereitungen sind Lösungen, Emulsionen oder injizierbare Lösungen, Infusionslösungen, Tropfen und als feste Zubereitungen Tabletten, Kapseln, Dragées, Pulver, Bolus, Pulverampullen zu nennen, die gebräuchliche Streckmittel, Verdünnungsmittel, Zusatzstoffe zur Beeinflussung des osmotischen Druckes, des pH-Wertes, des Geschmacks oder des Aromas, Gleitmittel, Benetzungsmittel, das Lösungsvermögen-retardierende Mittel enthalten können.

Die Erfindung betrifft somit auch pharmazeutische und Laboratoriums-Präparate, die als Wirkstoff 7-Oxo-$PGI_2$-derivate der allgemeinen Formel I enthalten, für die die Aggregation, die Thrombose- und die Magensekretion hemmende und antiasthmatische Behandlung.

Die weiteren Einzelheiten der Erfindung sind den folgenden Beispielen zu entnehmen.

*Beispiel 1*

(Herstellung des 6-Keto-$PGI_{1\alpha}$-derivats)

7$\beta$-Acetoxy-6-keto-$PGF_{1\alpha}$-methylester-methylketal (Verbindung der Formel IV; $R^5$ und Q = Methyl, $R^1$ und $R^2$ = Wasserstoff, X = trans-Vinylen, $R^4$ = $\beta$-Wasserstoff, Y = Methylen, $R^3$ = Propyl)

1 g der Verbindung der Formel III (worin die Substituenten die im vorhergehenden Absatz angegebene Bedeutung haben) löst man in 10 ml Methanol, gibt zur Lösung eine katalytische Menge Bortrifluoridätherat und rührt das Reaktionsgemisch 20 Minuten. Nach Zugabe einer kleinen Menge an festem Natriumhydrogencarbonat destilliert man das Methanol im Vakuum ab, löst den Rückstand in Äthylacetat, wäscht die Äthylacetatlösung zunächst mit Wasser, dann mit einer gesättigten wässrigen Natriumchloridlösung, trocknet sie über wasserfreiem Natriumsulfat, filtriert sie und destilliert das Lösungsmittel ab. Das als Rückstand erhaltene Öl wird an 120 g Silicagel chromatographiert und mit Äthylenacetat eluiert.

Man erhält 240 mg (22%) exo-Methylketal und 680 mg (63%) endo-Methylketal in Form eines farblosen Öls.

exo-IV: $R_f$: 0,31 Laufmittel: Äthylacetat

$\delta^1$H NMR 3,64 (3H, $COOCH_3$), 3,24 (3H, $OCH_3$)

endo-IV: $R_f$: 0,26 Laufmittel: Äthylacetat

$\delta^1$H NMR 3,57 (3H, $COOCH_3$), 3,14 (3H, $OCH_3$).

*Beispiel 2*

(Einführung der Schutzgruppen)

7$\beta$-Acetoxy-6-keto-$PGF_{1\alpha}$-methylester-methylketal-11,15-bis-(tert.-butyl-dimethyl-silyl)-äther (Verbindung der Formel V; $R^6$ = tert.-Butyl-dimethyl-silyl, die übrigen Substituenten, wie in der in Beispiel 1 erhaltenen Verbindung)

700 mg (1,52 mMol) des nach Beispiel 1 erhaltenen Methylketals der Formel IV, löst man in 1,4 ml wasserfreiem Dimethylformamid, gibt zur Lösung 517 mg (7,6 mMol) Imidazol und 552 mg (3,6 mMol) tert.-Butyl-dimethyl-chlor-silan, rührt das erhaltene Reaktionsgemisch 4 Stunden bei 35 bis 40°C und gibt es dann in 30 ml Wasser. Die wässrige Lösung extrahiert man 3 mal mit je 50 ml Dichlormethan, wäscht die vereinigten Dichlormethan-extrakte mit einer gesättigten wässrigen Natriumchloridlösung, trocknet sie mit wasserfreiem Natriumsulfat und destilliert das Lösungsmittel ab. Das als Rückstand erhaltene Öl wird an 120 g Silicagel chromatographiert und mit einem 8 : 1 Gemisch von Hexan-Äthylacetat eluiert.

Man erhält 788 mg (75%) des gewünschten Produktes in Form eines farblosen Öls.

$R_f$: 0,42, Laufmittel: ein 7 : 1 Gemisch von Hexan und Äthylacetat.

$\delta^1$H NMR: 2,05 (3H, OAc), 0,88 (6H, tert.-Butyl).

*Beispiel 3*

(7-Desacetylierung)

7$\beta$-Hydroxy-6-keto-$PGF_{1\alpha}$-methylester-methylketal-11,15-bis-(tert.-butyl-dimethyl-silyl)-äther (Verbindung der Formel IV; Substituenten, wie in der in Beispiel 2 erhaltenen Verbindung)

200 mg (0,29 mMol) des nach Beispiel 2 erhaltenen Silyläthers der Formel V löst man in 2 ml Methanol, gibt eine katalytische Menge frisch calciniertes Kaliumcarbonat zur Lösung, rührt das Reaktionsgemisch eine Stunde bei Raumtemperatur und destilliert das Lösungsmittel im Vakuum ab. Der Rückstand wird in Äthylacetat gelöst, mit Wasser und mit gesättigter wässriger Natriumchloridlösung gewaschen, mit wasserfreiem Natriumsulfat getrocknet, filtriert und dann das Lösungsmittel abdestilliert. Das als Rückstand erhaltene Öl wird an 20 g Silicagel chromatographiert und mit einem 4 : 1 Gemisch von Hexan und Äthylacetat eluiert.

Man erhält 150 mg (80%) des gewünschten Produktes in Form eines farblosen Öls.
$R_f$: 0,5 Laufmittel: ein 4 : 1 Gemisch von Hexan und Äthylacetat.
IR (Film) 335 0 $cm^{-1}$, (OH).

*Beispiel 4*
(7-Desacetylierung)

7β-Hydroxy-6-keto-$PGF_{1\alpha}$-methylester-hemiketal-11,15-bis-(tert.-butyl-dimethyl-silyl)-äther
(Verbindung der Formel VI; $R^5$ = Wasserstoff, die übrigen Substituenten, wie in der in Beispiel 2 erhaltenen Verbindung)

200 mg (0,29 mMol) des nach Beispiel 2 hergestellten Silyläthers der Formel V werden in 2 ml Methanol gelöst und der Lösung 50 mg frisch calciniertes Kaliumcarbonat zugegeben. Das Reaktionsgemisch wird bei Raumtemperatur 48 Stunden gerührt und wie in Beispiel 3 weiter aufgearbeitet. Das Rohprodukt wird an 20 g Silicagel chromatographiert und mit einem 2 : 1 Gemisch von Hexan und Äthylacetat eluiert.

Man erhält 113 mg (60%) des gewünschten Produktes in Form eines farblosen Öls.
$R_f$: 0,1 Laufmittel: ein 4 : 1 Gemisch von Hexan und Äthylacetat.
$\delta^1$H NMR: 3,67 (3H, $COOCH_3$).

*Beispiel 5*
(Oxydation der 7 OH-Gruppe)

7-Oxo-6-keto-$PGF_{1\alpha}$-methylester-methylketal-11,15-bis-(tert.-butyl-dimethyl-silyl)-äther
(Verbindung der Formel II; $R^5$ = Methyl, die übrigen Substituenten, wie in der in Beispiel 2 erhaltenen Verbindung)

75 mg (0,11 mMol) der Verbindung von Beispiel 3 (Formel VI) löst man in 0,7 ml Dichlormethan, gibt zur Lösung 63 mg (0,29 mMol) Pyridinium-chlorchromat, behandelt das Reaktionsgemisch mit einem Natriumacetatpuffer und rührt es fünf Stunden bei Raumtemperatur. Dann filtriert man das Reaktionsgemisch, wäscht es mit Dichlormethan und dampft die vereinigten Phasen ein. Der Rückstand wird an 10 g Silicagel mit einem 12 : 1 Gemisch von Hexan und Äthylacetat chromatographiert.

Man erhält 56 mg (75%) des gewünschten Produktes in Form eines farblosen Öls.
$R_f$: 0,59 Laufmittel: 7 : 1 Gemisch von Hexan und Äthylacetat.
IR (Film): 1735 $cm^{-1}$ (C=O), 1000-1250 $cm^{-1}$ (O—C—O).

*Beispiel 6*
(Oxydation der 7 OH-Gruppe)

7-Oxo-6-keto-$PGF_{1\alpha}$-methylester-hemiketal-11,15-bis-(tert.-butyl-dimethyl-silyl)-äther
(Verbindung der Formel II; $R^5$ = Wasserstoff, die übrigen Substituenten, wie in der in Beispiel 2 erhaltenen Verbindung)

50 mg (0,08 Mol) des nach Beispiel 4 hergestellten Silyläthers der Formel VI (worin $R^5$ = Wasserstoff ist) löst man in 0,5 ml Dichlormethan, gibt zur Lösung 45 mg (0,21 mMol) Pyridiniumchlorcarbonat, rührt das Reaktionsgemisch sechs Stunden bei Raumtemperatur und arbeitet weiter, wie in Beispiel 5 angegeben ist. Das Rohprodukt wird an 10 g Silicagel chromatographiert und mit einem 1 : 1 Gemisch von Äthylacetat-Hexan eluiert.

Man erhält 37 mg (75%) des gewünschten Produktes in Form eines farblosen Öls.
$R_f$: 0,55 Laufmittel: 1 : 1 Gemisch von Äthylacetat und Hexan.
IR (Film): 3300 $cm^{-1}$ (OH), 1730 $cm^{-1}$ (C=O).

*Beispiel 7*
(Einführung der Schutzgruppen)

7β-Acetoxy-6-keto-$PGF_{1\alpha}$-methylester-methylketal-11,15-bis-(tetrahydropyran-2-yl)-äther
(Verbindung der Formel V; $R^6$ = Tetrahydropyran-2-yl, die übrigen Substituenten, wie in der in Beispiel 1 erhaltenen Verbindung)

570 mg (1,24 mMol) der nach Beispiel 1 hergestellten Verbindung der Formel IV löst man in 5 ml wasserfreiem Dichlormethan, gibt zur Lösung 1,13 ml (12,4 mMol) wasserfreies 3,4-Dihydro-2H-pyran und eine katalytische Menge an p-Toluol-sulfonsäure, rührt das Reaktionsgemisch bei Raumtemperatur 10 Minuten und verdünnt es mit 50 ml Äthylacetat. Die Äthylacetatlösung wird mit gesättigter wässriger Natriumhydrogencarbonatlösung, mit Wasser und mit gesättigter Natriumchloridlösung gewaschen, über Natriumsulfat getrocknet, filtriert und danach das Lösungsmittel abdestilliert.

Man erhält 831 mg (Rohprodukt) des gewünschten Produktes in Form eines hellgelben Öls.
$R_f$: 0,53 Laufmittel: 1 : 1 Gemisch von Benzol und Äthylacetat.
IR (Film): 1740 $cm^{-1}$ (C=O), 100-1200 $cm^{-1}$ (O-CH-O).

Das erhaltene Produkt kann ohne Reinigung für die weitere Reaktion verwendet werden.

*Beispiel 8*
(7-Desacetylierung)

7β-Hydroxy-6-keto-$PGF_{1\alpha}$-methylester-methylketal-11,15-bis-(tetrahydropyran-2-yl)-äther
(Verbindung der Formel VI; Substituenten, wie in der in Beispiel 7 erhaltenen Verbindung)

831 mg (1,32 mMol) der in Beispiel 7 hergestellten Verbindung der Formel V löst man in 8 ml Methanol, gibt zur Lösung eine katalytische Menge frisch calciniertes Kaliumcarbonat, rührt das Reaktionsgemisch eine Stunde bei Raumtemperatur und führt die weitere Aufarbeitung wie in Beispiel 3 durch. Das als Rückstand erhaltene Öl wird an 40 g Silicagel mit einem 1 : 1 Gemisch von Benzol und Äthylacetat als Elutionsmittel chromatographiert.

Man erhält 471 mg (65%) des gewünschten Produktes in Form eines farblosen Öls.
$R_f$: 0,35 Laufmittel:1 : 1 Gemisch von Benzol und Äthylacetat.
IR (Film): 3350 $cm^{-1}$(OH), 1735 $cm^{-1}$ (C=O), 100-1200 $cm^{-1}$ (O-CH-O).

*Beispiel 9*
(Oxydation der 7 OH-Gruppe)

7-Oxo-6-keto-$PGF_{1\alpha}$-methylester-methyketal-11,15-bis-(tetrahydropyran-2-yl)-äther
(Verbindung der Formel II; die Substituenten, wie in der in Beispiel 7 erhaltenen Verbindung)

344 mg (0,59 mMol) der nach Beispiel 8 hergestellten Verbindung der Formel VI löst man in 3,5 ml Dichlormethan, gibt zur Lösung 254 mg (1,18 mMol) Pyridiniumchlorchromat, behandelt das Reaktionsgemisch mit Natriumacetatpuffer und rührt fünf Stunden bei Raumtemperatur. Die weitere Aufarbeitung des Reaktionsgemisches erfolgt nach der in Beispiel 5 angegebenen Weise. Das Rohprodukt wird an 30 g Silicagel mit einem 1 : 1 Gemisch von Benzol und Äthylacetat als Elutionsmittel chromatographiert.

Man erhält 275 mg (80%) des gewünschten Produktes als farbloses Öl.

$R_f$: 0,57 Laufmittel: ein 1 : 1 Gemisch von Benzol und Äthylacetat.

IR (Film): 1730-1740 $cm^{-1}$ (C=O), 1000-1200 $cm^{-1}$ (O-CH-O).

*Beispiel 10*
(Abspaltung der Schutzgruppen)

7-Oxo-6-keto-$PGF_{1\alpha}$-methylester-methylketal
(Verbindung der Formel II; $R^6$ = Wasserstoff, die übrigen Substituenten, wie in der in Beispiel 1 erhaltenen Verbindung)

194 mg (0,33 mMol) des nach Beispiel 9 erhaltenen Methylketals der Formel II (worin $R^6$ = Tetrahydropyranyl ist und die übrigen Substituenten die im vorangehenden Absatz angegebene Bedeutung haben) löst man in 10 ml Methanol, gibt zum Reaktionsgemisch eine katalytische Menge an p-Toluol-sulfonsäure, rührt die Lösung eine Stunde bei Raumtemperatur und destilliert das Lösungsmittel im Vakuum ab. Der Rückstand wird in Äthylacetat gelöst, die Äthylacetatlösung mit einer gesättigten Natriumhydrogencarbonatlösung, mit Wasser und dann mit wässriger Natriumchloridlösung gewaschen, über Natriumsulfat getrocknet, filtriert und dann das Lösungsmittel abdestilliert.

Das als Rückstand erhaltene Öl wird an 15 g Silicagel mit einem 2 : 1 Gemisch von Benzol und Äthylacetat chromatographiert.

Man erhält 117 mg (85%) des gewünschten Produktes in Form eines farblosen Öls.

$R_f$: 0,34 Laufmittel: Äthylacetat.

$\delta^1$H NMR ($CDCl_3$): 3,67 ($COOCH_3$), 3,17 ($OCH_3$)

IR (Film): 3350 $cm^{-1}$ (OH), 1735 $cm^{-1}$ (C=O).

Durch Verwendung von entsprechend substituierten Ausgangsverbindungen der allgemeinen Formel III erhält man nach den Verfahrensschritten der Beispiele 1 bis 10 die folgenden Zwischenprodukte der allgemeinen Formel II:

Hemiketale des 7-Oxo-6-keto-16-methyl-$PGF_{1\alpha}$-methyl-, -äthyl-, -propyl- und -butylester sowie deren Methyl-, Äthyl-, Propyl- und Butylketale.

Hemiketale des 7-Oxo-6-keto-16,16-dimethyl-$PGF_{1\alpha}$-methyl-, -äthyl-, -propyl- und -butylester sowie deren Methyl-, Äthyl-, Propyl- und Butylketale.

Hemiketale des 7-Oxo-6-keto-16-phenoxy-17,18,-19,20-tetra-nor-$PGF_{1\alpha}$-methyl-, -äthyl-, -propyl- und -butylester sowie deren Methyl-, Äthyl-, Propyl- und Butylketale.

Hemiketale des 7-Oxo-6-keto-20-methyl-$PGF_{1\alpha}$-methyl-, -äthyl-, -propyl- und -butylester sowie deren Methyl-, Äthyl-, Propyl- und Butylketale.

Hemiketale des 7-Oxo-6-keto-13,14-didehydro-$PGF_{1\alpha}$-methyl-, -äthyl-, -propyl- und -butylester sowie deren Methyl-, Äthyl-, Propyl- und Butylketale.

Hemiketale des 7-Oxo-6-keto-15-methyl-$PGF_{1\alpha}$-methyl-, -äthyl-, -propyl- und -butylester sowie deren Methyl-, Äthyl-, Propyl- und Butylketale.

Hemiketale des 7-Oxo-6-15-epi-$PGF_{1\alpha}$-methyl-, -äthyl-, -propyl- und -butylester sowie deren Methyl-, Äthyl-, Propyl- und Butylketale.

Hemiketale des 7-Oxo-6-keto-15-epi-16,16-dimethyl-$PGF_{1\alpha}$-methyl-, -äthyl-, -propyl- und -butylester sowie deren Methyl-, Äthyl-, Propyl- und Butylketale.

Hemiketale des 7-Oxo-6-keto-15-epi-16-phenoxy-17,18,19,20-tetra-nor-$PGF_{1\alpha}$-methyl-, -äthyl-, -propyl- und -butylester sowie deren Methyl-, Äthyl-, Propyl- und Butylketale.

Hemiketale des 7-Oxo-6-keto-15-epi-20-methyl-$PGF_{1\alpha}$-methyl-, -äthyl-, -propyl- und -butylester sowie deren Methyl-, Äthyl-, Propyl- und Butylketale.

Werden während der Synthese die Schutzgruppen gemäss Beispiel 10 nicht entfernt, werden die 11,15-bis-Silyloxy-, 11,15-bis-(Tetrahydropyran-2-yl-oxy)- und 11,15-bis-($\alpha$-Alkoxyalkyl)-derivate der oben angegebenen Verbindungen erhalten.
($R^6$ = Silyl, Tetrahydropyran-2-yl, $\alpha$-Alkoxyalkyl).

Unter «epi»-Derivat ist ein Derivat zu verstehen, in dem das durch die vor «epi» stehende Nummer gekennzeichnete Kohlenstoffatom einen Substituenten trägt, dessen Konfiguration der des Substituenten am entsprechenden Kohlenstoffatome der natürlichen Prostaglandine entgegengesetzt ist.

*Beispiel 11*
(Bildung der 5,6-Doppelbindung)

7-Oxo-$PGI_2$-methylester
(Verbindung der Formel I; Q = Methyl, $R^1$, $R^2$, $R^{13}$ = Wasserstoff, X = trans-Vinylen, $R^4$ = $\beta$-Wasserstoff, Y = Methylen und $R^3$ = Propyl)

20 mg (0,31 mMol) einer nach Beispiel 5 hergestellten Verbindung der Formel II (worin $R^5$ = Methyl, $R^6$ = Dimethyl-tert.-butyl-silyl und die übrigen Substituenten die im vorhergehenden Absatz angegebene Bedeutung haben) löst man in 5 ml Hexamethylphosphorsäuretriamid, rührt das Reaktionsgemisch drei Stunden bei 150 bis 160°C, giesst es in 18 ml Wasser, extrahiert die wässrige Phase 3 mal mit je 15 ml Äthylacetat, wäscht die vereinigten Äthylacetat-Extrakte mit gesättigter Natriumchloridlösung, trocknet sie mit wasserfreiem Natriumsulfat, filtriert und destilliert das Lösungsmittel ab. Das als Rückstand erhaltene Produkt, 250 mg, wird in 5 ml Tetrahydrofuran gelöst, und das nach Zugabe von 2 Äquivalenten des Tetrabutylammonium-fluorids erhaltene Gemisch wird bei Raumtemperatur drei Stunden gerührt. Das Lösungsmittel wird im Vakuum abdestilliert und der Rückstand in Äthylacetat

gelöst. Die erhaltene Äthylacetatlösung wird mit Wasser, dann mit gesättigter wässriger Natriumchloridlösung gewaschen, über wasserfreiem Natriumsulfat getrocknet, filtriert und schliesslich wird das Lösungsmittel abdestilliert. Das als Rückstand erhaltene Öl wird an 10 g Silicagel mit Äthylacetat chromatographiert.

Man erhält 62 ml (50%) des gewünschten Produktes in Form eines farblosen Öls.

$R_f$: 0,44 Laufmittel: Äthylacetat.

$\delta^1H$ NMR: 5,37 (1H, t, O-C=CH-), 3,76 (3H, s, $-COOCH_3$).

*Beispiel 12*
(Bildung der 5,6-Doppelbindung)

7-Oxo-$PGI_2$-methylester

200 mg (0,31 mMol) der nach Beispiel 6 hergestellten Verbindung der allgemeinen Formel II ($R^5$ = Wasserstoff, $R^6$ = Dimethyl-tert.-butyl-silyl, die übrigen Substituenten, wie in der in Beispiel 11 erhaltenen Verbindung) erhitzt man in 30 ml wasserfreiem Benzol und in einem wasserfreies Magnesiumsulfat enthaltenden Soxhlet-Extraktor zwei Stunden zum Sieden. Die organische Phase wird zunächst mit Wasser, dann mit gesättigter wässriger Natriumchloridlösung gewaschen, mit wasserfreiem Natriumsulfat getrocknet, filtriert, das Lösungsmittel abdestilliert und das erhaltene Rohprodukt in Tetrahydrofuran mit 2 Äquivalenten Tetrabutylammonium-fluorid behandelt. Die weitere Aufarbeitung des Reaktionsgemisches erfolgt in der in Beispiel 11 angegebenen Weise. Das Gemisch wird an 10 g Silicagel mit Äthylacetat chromatographiert.

Man erhält 52 mg (45%) des gewünschten Produktes in Form eines farblosen Öls.

Die physikalischen Konstanten des Produktes stimmen mit denen des Beispiels 11 überein.

*Beispiel 13*
(Bildung der 5,6-Doppelbindung)

7-Oxo-$PGI_2$-methylester

90 mg (0,215 mMol) des nach Beispiel 10 erhaltenen 7-Oxo-6-keto-$PGF_{1\alpha}$-methylester-methylketals der Formel II ($R^6$ = $R^{13}$ = Wasserstoff) werden in 0,8 ml Hexamethyl-phosphorsäure-triamid gelöst und das Gemisch zwei Stunden bei 140 bis 150°C gerührt. Das Reaktionsgemisch wird wie in Beispiel 11 weiter aufgearbeitet. Das als Rückstand erhaltene Öl wird an 10 g Silicagel mit Äthylacetat chromatographiert.

Man erhält 40 mg (47%) des gewünschten Produktes in Form eines farblosen Öls.

Die physikalischen Konstanten des Produktes stimmen mit denen des Beispiels 11 überein.

*Beispiel 14*
(Bildung der 5,6-Doppelbindung)

7-Oxo-$PGI_2$-methylester-11,15-diacetat
(Verbindung der Formel I; Q = Methyl, $R^1$, $R^2$ = Wasserstoff, $R^{13}$ = Acetyl, $R^4$ = $\beta$-Wasserstoff, X = trans-Vinylen und Y = Methylen)

100 mg 7-Oxo-6-keto-$PGF_{1\alpha}$-methylester-methylketal-11,15-diacetat werden in 3 ml Hexamethylphosphorsäure-triamid gelöst und in Gegenwart von 0,05 ml Essigsäureanhydrid zwei Stunden bei 80 bis 100°C gerührt. Die weitere Aufarbeitung des Reaktionsgemisches erfolgt wie in Beispiel 11. Das Rohprodukt wird an 15 g Silicagel mit einem 1 : 1 Gemisch von Äthylacetat und Hexan chromatographiert.

Man erhält 70 mg (72%) eines Produktes in Form eines farblosen Öls.

$R_f$: 0,65 Laufmittel: 1 : 1 Gemisch von Äthylacetat und Hexan.

*Beispiel 15*
(Bildung der 5,6-Doppelbindung)

16,16-Dimethyl-7-oxo-$PGI_2$-methylester
(Verbindung der Formel I; $R^1$ und $R^2$ = Methyl, die übrigen Substituenten, wie die in Beispiel 11 erhaltene Verbindung)

90 mg (0,21 mMol) 16,16-Dimethyl-7-oxo-6-keto-$PGF_{1\alpha}$-methylester-methylketal ($R^1$ und $R^2$ = Methyl) werden in 1 ml Hexamethyl-phosphorsäure-triamid gelöst und das Reaktionsgemisch zwei Stunden bei 140 bis 150°C gerührt. Die weitere Aufarbeitung erfolgt in der in Beispiel 13 angegebenen Weise.

Man erhält 50 mg (56%) der Gewünschten Verbindung in Form eines farblosen Öls.

$R_f$: 0,52 (in Äthylacetat).

*Beispiel 16*
(Salzbildung)

7-Oxo-$PGI_2$-Natriumsalz
(Verbindung der Formel I; Q = Natrium, die übrigen Substituenten, wie in der in Beispiel 11 erhaltenen Verbindung)

50 mg (0,13 mMol) 7-Oxo-$PGI_2$-methylester werden in 0,1 ml Methanol gelöst und das Gemisch nach Zugabe von 1,4 ml 0,1 n wässriger Natriumhydroxydlösung bei Raumtemperatur 24 Stunden gerührt. Die Lösung wird lyophilisiert.

Man erhält 55 mg des gewünschten Produktes in Form einer weissen Masse.

Unter Anwendung der Umsetzungen der Beispiele 11 bis 16 sind aus den nach den Beispielen 1 bis 10 hergestellten Verbindungen der Formel II die folgenden Verbindungen erhalten worden:

7-Oxo-16-phenoxy-17,18,19,20-tetra-nor-$PGI_2$ und dessen $C_{1-4}$-Alkylester ($R_f$ des Methylesters in Äthylacetat: 0,57).

7-Oxo-16-methyl-$PGI_2$ und dessen $C_{1-4}$-Alkylester ($R_f$ des Methylesters in Äthylacetat: 0,49).

7-Oxo-20-methyl-$PGI_2$ und dessen $C_{1-4}$-Alkylester ($R_f$ des Methylesters in Äthylacetat: 0,51).

7-Oxo-13,14-didehydro-$PGI_2$ und dessen $C_{1-4}$-Alkylester ($R_f$ des Methylesters in Äthylacetat: 0,47).

7-Oxo-15-methyl-$PGI_2$ und dessen $C_{1-4}$-Alkylester ($R_f$ des Methylesters in Äthylacetat: 0,53).

7-Oxo-15-epi-$PGI_2$ und dessen $C_{1-4}$-Alkylester ($R_f$ des Methylesters in Äthylacetat: 0,50).

7-Oxo-15-epi-16,16-dimethyl-$PGI_2$ und dessen $C_{1-4}$-Alkylester ($R_f$ des Methylesters in Äthylacetat: 0,58).

7-Oxo-15-epi-16-phenoxy-17,18,19,20-tetra-nor--$PGI_2$ und dessen $C_{1-4}$-Alkylester ($R_f$ des Methylesters in Äthylacetat: 0,63).

7-Oxo-15-epi-20-methyl-$PGI_2$ und dessen $C_{1-4}$-Alkylester ($R_f$ des Methylesters in Äthylacetat: 0,55).

Die freien Säuren können durch die in Beispiel 16 angegebene Verseifung aus dem Alkalimetallsalz, z.B. dem Natriumsalz, hergestellt werden, indem man dessen wässrige Lösung mit einer starken organischen oder anorganischen Säure ansäuert. Die freie Säure scheidet sich aus der Lösung aus.

**Patentansprüche**

1. Optisch aktive und racemische 7-Oxo--$PGI_2$-derivate der allgemeinen Formel

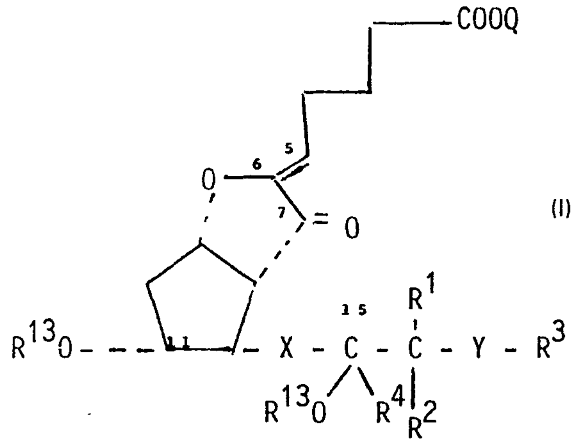

(I)

worin

Q Wasserstoff, ein physiologisch verträgliches Kation oder $C_{1-6}$-Alkyl,

X cis- oder trans-CH=CH, -C≡C- oder $-CH_2-CH_2-$,

$R^{13}$ Wasserstoff, $C_{1-4}$-Alkanoyl, eine Schutzgruppe der allgemeinen Formel $R^7R^8R^9Si$- oder $R^{11}CH_2-C(R^{10})(OR^{12})$- bedeuten,

worin $R^7$, $R^8$ und $R^9$, die gleich oder verschieden sein können, $C_{1-4}$-Alkyl,

$R^{10}$ und $R^{11}$, die gleich oder verschieden sein können, Wasserstoff oder Methyl und

$R^{12}$ Methyl oder Äthyl bedeuten oder die Tetrahydro-pyran-2-yl-gruppe,

$R^4$ Wasserstoff, $C_{1-6}$-Alkyl in sterischer $\alpha$- oder $\beta$-Konfiguration,

$R^1$ und $R^2$ Wasserstoff oder $C_{1-6}$-Alkyl,

Y Methylen, Sauerstoff oder -NH- und

$R^3$ $C_{1-6}$-Alkyl oder Phenyl, das durch Halogen substituiertes $C_{1-6}$-Alkyl, Hydroxyl, Sulfhydryl, $C_{1-6}$-Alkanoyloxy, Benzoyloxy, Amino, Mono- oder Di-$C_{1-6}$-alkylamino, Halogen, $C_{1-6}$-Alkoxy oder $C_{1-6}$-Alkylthio monosubstituiert sein kann, bedeuten.

2. 7-Oxo-$PGI_2$-methylester.

3. 7-Oxo-$PGI_2$-methylester-11,15-diacetat.

4. 16,16-Dimethyl-7-oxo-$PGI_2$-methylester.

5. 7-Oxo-$PGI_2$-Natriumsalz.

6. 7-Oxo-16-phenoxy-17,18,19,20-tetra-nor--$PGI_2$-methylester.

7. 7-Oxo-16-methyl-$PGI_2$-methylester.

8. 7-Oxo-20-methyl-$PGI_2$-methylester.

9. 7-Oxo-13,14-didehydro-$PGI_2$-methylester.

10. 7-Oxo-15-methyl-$PGI_2$-methylester.

11. 7-Oxo-15-epi-$PGI_2$-methylester.

12. 7-Oxo-15-epi-16,16-dimethyl-17,18,19,20--tetra-nor-$PGI_2$-methylester.

13. 7-Oxo-15-epi-16-phenoxy-17,18,19,20-tetra-nor-$PGI_2$-methylester.

14. 7-Oxo-15-epi-20-methyl-$PGI_2$-methylester.

15. Verfahren zur Herstellung von optisch aktiven oder racemischen 7-Oxo-$PGI_2$-derivaten der allgemeinen Formel

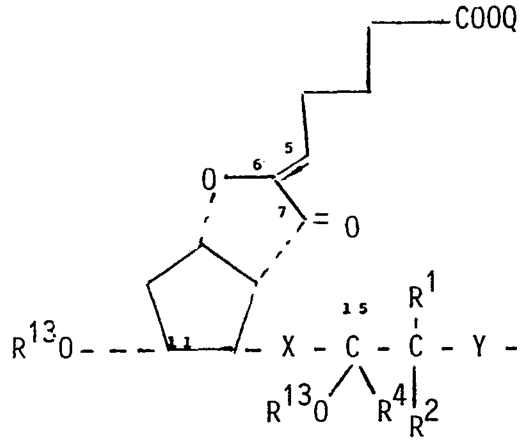

(I)

worin

Q Wasserstoff, ein physiologisch verträgliches Kation oder $C_{1-6}$-Alkyl,

X cis- oder trans-CH=CH, -C≡C- oder $-CH_2-CH_2-$,

$R^{13}$ Wasserstoff, $C_{1-4}$-Alkanoyl, eine Schutzgruppe der allgemeinen Formel $R^7R^8R^9Si$- oder $R^{11}CH_2-C(R^{10})(OR^{12})$- bedeuten,

worin $R^7$, $R^8$ und $R^9$, die gleich oder verschieden sein können, $C_{1-4}$-Alkyl,

$R^{10}$ und $R^{11}$, die gleich oder verschieden sein können, Wasserstoff oder Methyl und

$R^{12}$ Methyl oder Äthyl bedeuten oder die Tetrahydro-pyran-2-yl-gruppe,

$R^4$ Wasserstoff, $C_{1-6}$-Alkyl in sterischer $\alpha$- oder $\beta$-Konfiguration,

$R^1$ und $R^2$ Wasserstoff oder $C_{1-6}$-Alkyl,

Y Methylen, Sauerstoff oder -NH- und

$R^3$ $C_{1-6}$-Alkyl oder Phenyl, das durch Halogen substituiertes $C_{1-6}$-Alkyl, Hydroxyl, Sulfhydryl, $C_{1-6}$-Alkanoyloxy, Benzoyloxy, Amino, Mono- oder Di-$C_{1-6}$-alkylamino, Halogen, $C_{1-6}$-Alkoxy oder $C_{1-6}$-Alkylthio monosubstituiert sein kann, bedeuten,

dadurch gekennzeichnet, dass man eine Verbindung der allgemeinen Formel

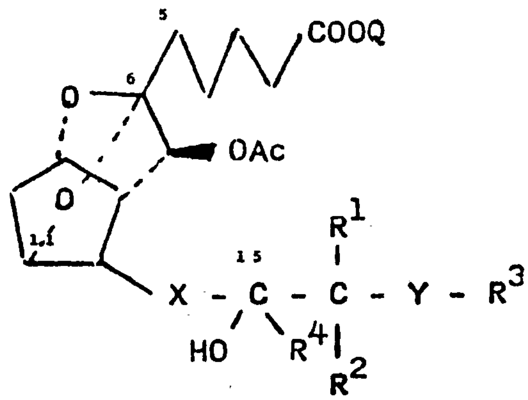

(III)

worin $R^1$, $R^2$, $R^3$, $R^4$, X, Y und Q die oben angegebene Bedeutung haben und

Ac Acetyl ist,

mit einem niederen Alkanol in Gegenwart einer katalytischen Menge eines Säurekatalysators umsetzt, in den erhaltenen 6-Ketalen der allgemeinen Formel

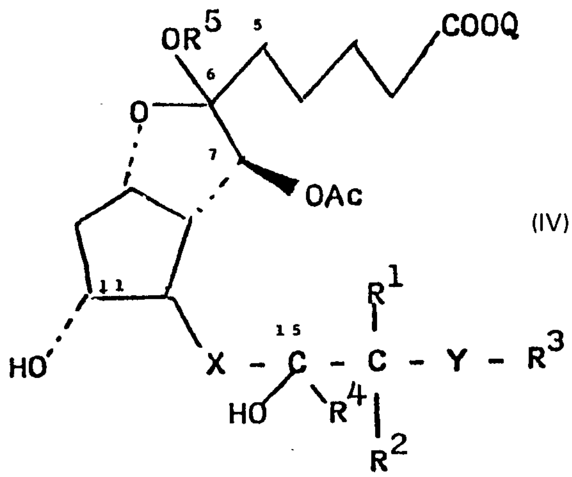

(IV)

worin $R^1$, $R^2$, $R^3$, $R^4$, X, Y, Q und Ac die oben angegebene Bedeutung haben und

$R^5$ $C_{1-6}$-Alkyl ist,

die 11- und 15-Hydroxylgruppen durch Umsetzung mit einem Trialkylchlorsilan der allgemeinen Formel

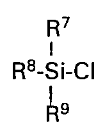

(VII)

einem Enol-äther der allgemeinen Formel

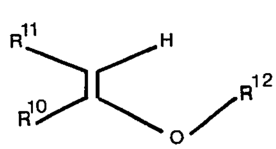

(VIII)

worin $R^7$, $R^8$, $R^9$, die gleich oder verschieden sein können, $C_{1-4}$-Alkyl,

$R^{10}$ und $R^{11}$, die gleich oder verschieden sein können, Wasserstoff oder Methyl und

$R^{12}$ Methyl oder Äthyl bedeuten

oder mit

3,4-Dihydro-2H-pyran

schützt und die erhaltenen Ketale der allgemeinen Formel

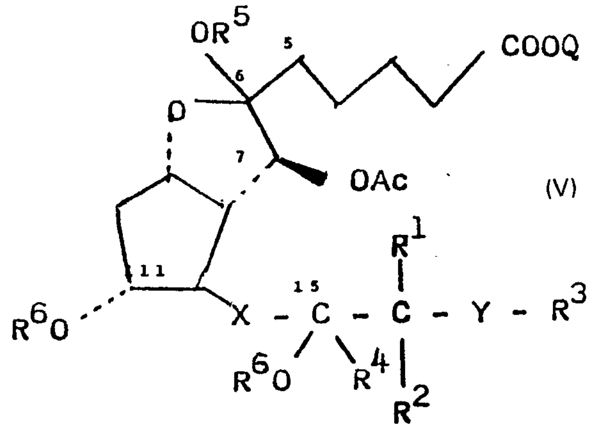

(V)

worin $R^1$, $R^2$, $R^3$, $R^4$, $R^5$, X, Y, Q und Ac die oben angegebene Bedeutung haben und

$R^6$ eine Silylgruppe der Formel $R^7R^8R^9Si$-, eine α-Alkoxyalkyl- der Formel $R^{11}CH_2-C(R^{10})(OR^{12})$-

oder eine Tetrahydropyran-2-yl-gruppe bedeutet,

in 7-Stellung in basischem Medium desacetyliert, die gebildete freie Hydroxylgruppe in 7-Stellung der erhaltenen geschützten Ketale der allgemeinen Formel

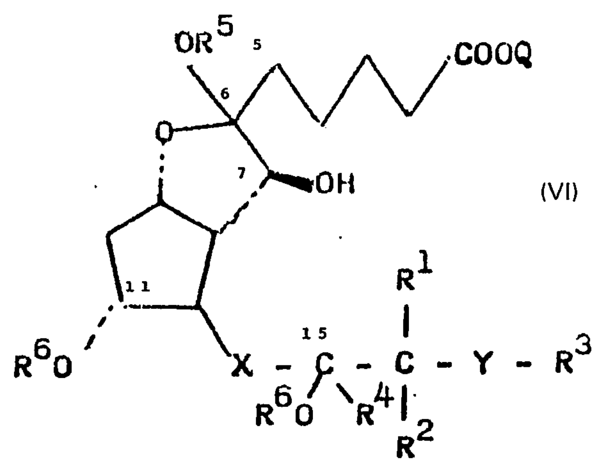

(VI)

worin $R^1$, $R^2$, $R^3$, $R^4$, $R^5$, $R^6$, X, Y und Q die oben angegebene Bedeutung haben,

gegebenenfalls nach Entfernung der $R^5$-Alkylgruppe durch Hydrolyse in aprotischen Lösungsmitteln, oxydiert und die erhaltenen 7-Oxo-derivate der allgemeinen Formel

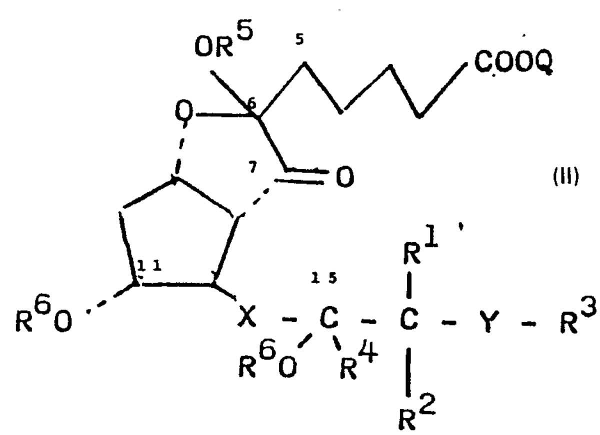

(II)

worin $R^1$, $R^2$, $R^3$, $R^4$, $R^5$, $R^6$, X, Y und Q die oben angegebene Bedeutung haben,
gegebenenfalls nach Abspalten der Schutzgruppen $R^6$, das von einer Acylierung der bei der $R^6$-Schutzgruppenabspaltung gebildeten freien 11- und 15-Hydroxylgruppen mit niederem Alkanoyl gefolgt sein kann,
wenn $R^5$ $C_{1-6}$-Alkyl ist, durch Abspalten eines Alkohols der Formel $R^5$-OH,
wenn $R^5$ durch Wasserstoff ersetzt ist, durch Abspalten von Wasser,
in eine Verbindung der allgemeinen Formel I überführt, in der gewünschtenfalls die Schutzgruppen $R^{13}$ abgespalten und/oder die Estergruppen verseift und/oder die freie Säure in ein Salz oder einen Ester übergeführt wird.

16. Pharmazeutische Präparate, die als Wirkstoff ein oder mehrere der 7-Oxo-$PGI_2$-derivate der Ansprüche 1 bis 14 enthalten.

## Claims

1. Optically active and racemic 7-oxo-$PGI_2$derivatives of general formula

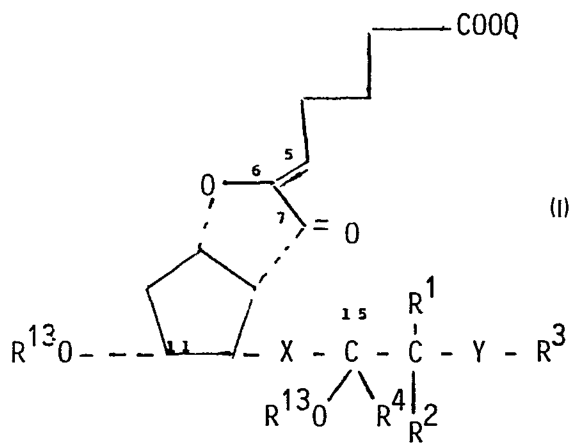

(I)

wherein
Q represents hydrogen, a physiologically acceptable cation or $C_{1-6}$ alkyl,
X represents cis or trans -CH=CH-, -C≡C- or $-CH_2-CH_2-$,
$R^{13}$ represents hydrogen, $C_{1-4}$ alkanoyl, a protecting group of general formula
$R^7R^8R^9Si$- or $R^{11}CH_2-C(R^{10})-$, $OR^{12}$
wherein $R^7$, $R^8$ and $R^9$, which may be identical or different, represent $C_{1-4}$ alkyl,
$R^{10}$ and $R^{11}$, which may be identical or different represent hydrogen or methyl and
$R^{12}$ represents methyl or ethyl, or the tetrahydropyran-2-yl group,
$R^4$ represents hydrogen, $C_{1-6}$ alkyl in the steric $\alpha$ or $\beta$ configuration,
$R^1$ and $R^2$ represent hydrogen or $C_{1-6}$ alkyl,
Y represents methylene, oxygen or -NH- and
$R^3$ represents $C_{1-6}$ alkyl or phenyl which may be monosubstituted by halogen-substituted $C_{1-6}$ alkyl, hydroxyl, mercapto, $C_{1-6}$ alkanoyloxy, benzoyloxy, amino, mono- or di-$C_{1-6}$-alkylamino, halogen, $C_{1-6}$ alkoxy or $C_{1-6}$ alkylthio.

2. 7-Oxo-$PGI_2$-methyl ester.
3. 7-Oxo-$PGI_2$-methyl ester-11,15-diacetat.
4. 16,16-Dimethyl-7-oxo-$PGI_2$-methyl ester.
5. Sodium salt of 7-oxo-$PGI_2$.
6. 7-Oxo-16-phenoxy-17,18,19,20-tetra-nor-$PGI_2$-methyl ester.
7. 7-Oxo-16-methyl-$PGI_2$-methyl ester.
8. 7-Oxo-20-methyl-$PGI_2$-methyl ester.
9. 7-Oxo-13,14-didehydro-$PGI_2$-methyl ester.
10. 7-Oxo-15-methyl-$PGI_2$-methyl ester.
11. 7-Oxo-15-epi-$PGI_2$-methyl ester.
12. 7-Oxo-15-epi-16,16-dimethyl-17,18,19,20-tetra-nor-$PGI_2$-methyl ester.
13. 7-Oxo-15-epi-16-phenoxy-17,18,19,20-tetra-nor-$PGI_2$-methyl ester.
14. 7-Oxo-15-epi-20-methyl-$PGI_2$-methyl ester.
15. Process for preparing optically active or racemic 7-oxo-$PGI_2$ derivatives of general formula

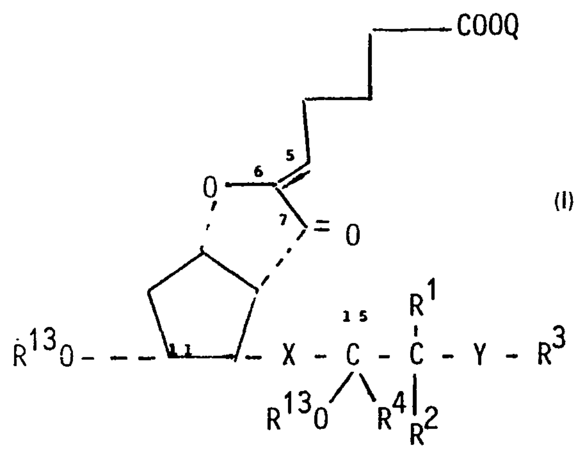

(I)

wherein
Q represents hydrogen, a physiologically acceptable cation or $C_{1-6}$ alkyl,
X represents cis or trans -CH=CH-, -C≡C- or $-CH_2-CH_2-$,
$R^{13}$ represents hydrogen, $C_{1-4}$ alkanoyl, a protecting group of general formula
$R^7R^8R^9Si$- or $R^{11}CH_2-C(R^{10})-$, $OR^{12}$
wherein $R^7$, $R^8$ and $R^9$, which may be identical or different, represent $C_{1-4}$ alkyl,
$R^{10}$ and $R^{11}$, which may be identical or different represent hydrogen or methyl and
$R^{12}$ represents methyl or ethyl, or the tetrahydropyran-2-yl group,
$R^4$ represents hydrogen, $C_{1-6}$ alkyl in the steric $\alpha$ or $\beta$ configuration,
$R^1$ and $R^2$ represent hydrogen or $C_{1-6}$ alkyl,
Y represents methylene, oxygen or -NH- and
$R^3$ represents $C_{1-6}$ alkyl or phenyl which may be monosubstituted by halogen-substituted $C_{1-6}$ alkyl, hydroxyl, mercapto, $C_{1-6}$ alkanoyloxy, benzoyloxy, amino, mono- or di-$C_{1-6}$-alkylamino, halogen, $C_{1-6}$ alkoxy or $C_{1-6}$ alkylthio,
characterized in that a compound of general formula

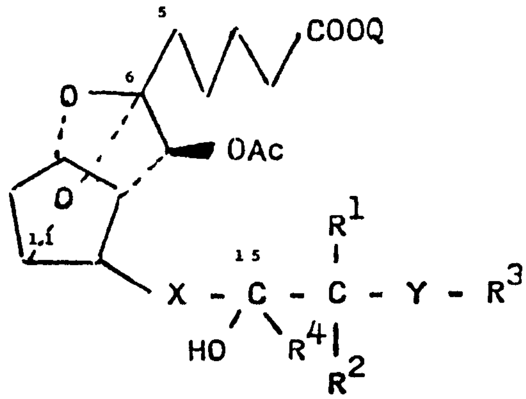

(III)

wherein $R^1$, $R^2$, $R^3$, $R^4$, X, Y and Q are as hereinbefore defined and

Ac is acetyl,

is reacted with a lower alkanol in the presence of a catalytic quantity of an acid catalyst, and in the resulting 6-ketals of general formula

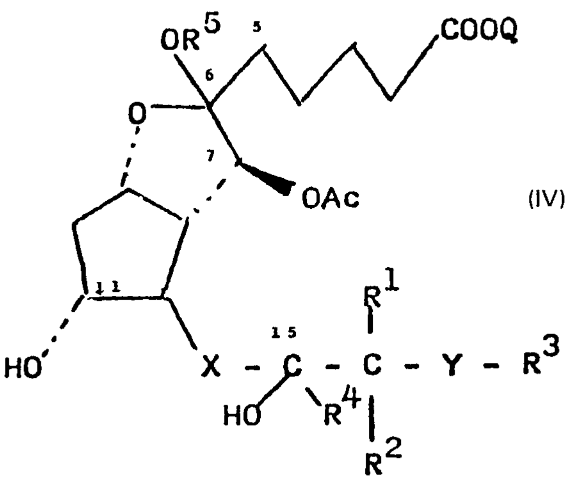

(IV)

wherein $R^1$, $R^2$, $R^3$, $R^4$, X, Y, Q and Ac are as hereinbefore defined and

$R^5$ represents $C_{1-6}$ alkyl,

the 11- and 15-hydroxyl groups are protected by reacting with a trialkylchlorsilane of general formula

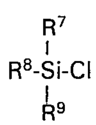

(VII)

an enol ether of general formula

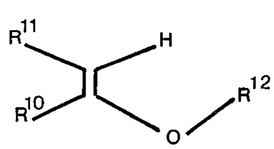

(VIII)

wherein $R^7$, $R^8$ and $R^9$, which may be identical or different, represent $C_{1-4}$ alkyl,

$R^{10}$ and $R^{11}$, which may be identical or different, represent hydrogen or methyl and

$R^{12}$ represents methyl or ethyl,

or with 3,4-dihydro-2H-pyran,

and the resulting ketals of general formula

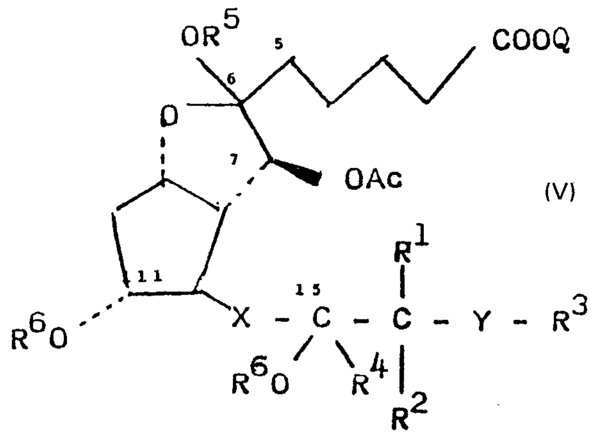

(V)

wherein $R^1$, $R^2$, $R^3$, $R^4$, $R^5$, X, Y, Q and Ac are as hereinbefore defined and

$R^6$ represents a silyl group of formula $R^7R^8R^9Si$-, an $\alpha$-alkoxyalkyl of formula $R^{11}CH_2$-$C(R^{10})(OR^{12})$-

or a tetrahydropyran-2-yl group,

are deacetylated in the 7 position in a basic medium, and the free hydroxyl group formed in the 7 position of the resulting pretected ketals of general formula

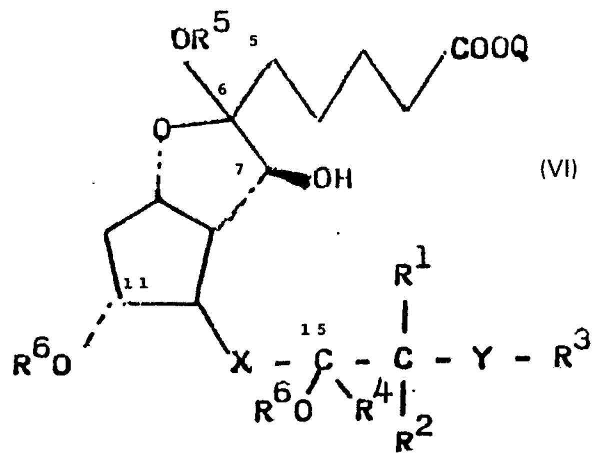

(VI)

wherein $R^1$, $R^2$, $R^3$, $R^4$, $R^5$, $R^6$, X, Y and Q are as hereinbefore defined,

is oxidized, optionally after removal of the $R^5$-alkyl group by hydrolysis in aprotic solvents, and the resulting 7-oxo derivatives of general formula

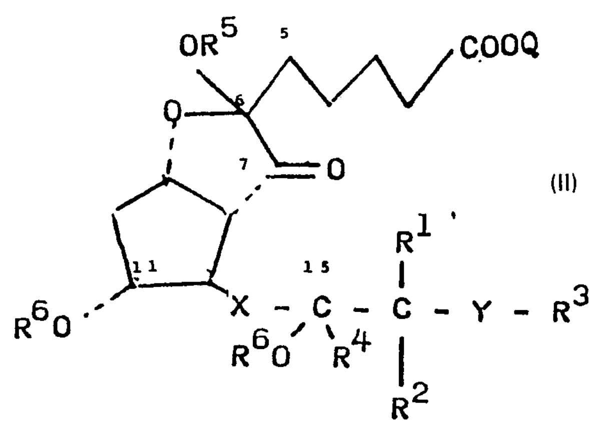

(II)

wherein $R^1$, $R^2$, $R^3$, $R^4$, $R^5$, $R^6$, X, Y and Q are as hereinbefore defined,

optionally after the protecting groups $R^6$ have been split off, optionally followed by acylation of the free 11- and 15-hydroxyl groups formed during the splitting off of the $R^6$ protecting groups, with a lower alkanoyl,
if $R^5$ represents $C_{1-6}$ alkyl, by splitting off an alcohol of formula $R^5$-OH,
if $R^5$ is replaced by hydrogen, by splitting off water, are converted into compounds of general formula I wherein, if desired, the protecting groups $R^{13}$ are split off and/or the ester groups are saponified and/or the free acid is converted into a salt or an ester.

16. Pharmaceutical preparations which contain, as active substance, one or more of the 7-oxo-$PGI_2$ derivatives as claimed in claims 1 to 14.

**Revendications**

1. Dérivés de 7-oxo-$PGI_2$ optiquement actifs et racémiques de formule générale:

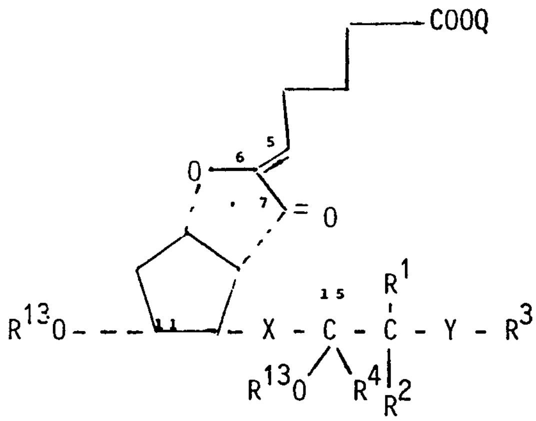

(I)

où Q représente un hydrogène, un cation physiologiquement acceptable ou un alcoyle en $C_1$ à $C_6$; X représente un cis- ou trans-$CH=CH$-, $-C\equiv C-$ ou $-CH_2-CH_2-$; $R^{13}$ représente un hydrogène, un alcanoyle en $C_1$ à $C_4$, un groupe protecteur de formule générale:

$$R^7R^8R^9Si-$$

ou

$$R^{11}CH_2-\underset{OR^{12}}{C}(R^{10})-$$

où $R^7$, $R^8$ et $R^9$ qui peuvent être semblables ou différents, représentent un alcoyle en $C_1$ à $C_4$; $R^{10}$ et $R^{11}$, qui peuvent être semblables ou différents, représentent un hydrogène ou un méthyle; $R^{12}$ représente un méthyle ou un éthyle ou le groupe tétrahydropyran-2-yl; $R^4$ représente un hydrogène, un alcoyle en $C_1$ à $C_6$ en configuration stérique $\alpha$ ou $\beta$; $R^1$ et $R^2$ représentent un hydrogène ou un alcoyle en $C_1$ à $C_6$; Y représente un méthylène, un oxygène ou -NH- et $R^3$ représente un alcoyle en $C_1$ à $C_6$ ou un phényle, qui peut être monosubstitué par un alcoyle en $C_1$ à $C_6$ substitué par un halogène, un hydroxyle, un sulfhydryle, un alcanoyloxy en $C_1$ à $C_6$, un benzoyloxy, un amino, un mono- ou un di-alcoyle en $C_1$ à $C_6$-amino, un halogène, un alcoxy en $C_1$ à $C_6$ ou un alcoylthio en $C_1$ à $C_6$.

2. 7-oxo-$PGI_2$-méthylester.
3. 7-oxo-$PGI_2$-méthylester-11,15-diacétate.
4. 16,16-diméthyl-7-oxo-$PGI_2$-méthylester.
5. Sel de sodium de 7-oxo-$PGI_2$.
6. 7-oxo-16-phénoxy-17,18,19,20-tétra-nor-$PGI_2$-méthylester.
7. 7-oxo-16-méthyl-$PGI_2$-méthylester.
8. 7-oxo-20-méthyl-$PGI_2$-méthylester.
9. 7-oxo-13,14-didéshydro-$PGI_2$-méthylester.
10. 7-oxo-15-méthyl-$PGI_2$-méthylester.
11. 7-oxo-15-épi-$PGI_2$-méthylester.
12. 7-oxo-15-épi-16,16-diméthyl-17,18,19,20-tétra-nor-$PGI_2$-méthylester.
13. 7-oxo-15-épi-16-phénoxy-17,18,19,20-tétra-nor-$PGI_2$-méthylester.
14. 7-oxo-15-épi-20-méthyl-$PGI_2$-méthylester.
15. Procédé de préparation de dérivés de 7-oxo-$PGI_2$ optiquement actifs ou racémiques de formule générale:

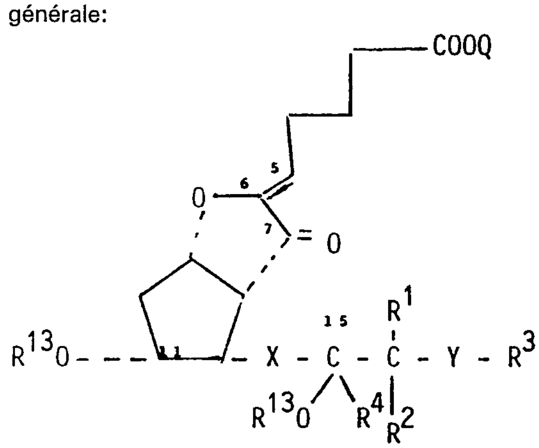

(I)

où Q représente un hydrogène, un cation physiologiquement acceptable ou un alcoyle en $C_1$ à $C_6$; X représente un cis- ou trans-$CH=CH$-, $-C\equiv C-$ ou $-CH_2-CH_2-$; $R^{13}$ représente un hydrogène, un alcanoyle en $C_1$ à $C_4$, un groupe protecteur de formule générale:

$$R^7R^8R^9Si-$$

ou

$$R^{11}CH_2-\underset{OR^{12}}{C}(R^{10})-$$

où $R^7$, $R^8$ et $R^9$ qui peuvent être semblables ou différents, représentent un alcoyle en $C_1$ à $C_4$; $R^{10}$ et $R^{11}$, qui peuvent être semblables ou différents, représentent un hydrogène ou un méthyle; $R^{12}$ représente un méthyle ou un éthyle ou le groupe tétrahydropyran-2-yl; $R^4$ représente un hydrogène, un alcoyle en $C_1$ à $C_6$ en configuration stérique $\alpha$ ou $\beta$; $R^1$ et $R^2$ représentent un hydrogène ou un alcoyle en $C_1$ à $C_6$; Y représente un méthylène, un oxygène ou -NH- et $R^3$ représente un alcoyle en $C_1$ à $C_6$ ou un phényle, qui peut être monosubstitué par un alcoyle en $C_1$ à $C_6$ substitué par un halogène, un hydroxyle, un sulfhydryle, un alcanoyloxy en $C_1$ à $C_6$, un benzoyloxy, un amino, un mono- ou un di-alcoyle en $C_1$ à $C_6$-amino, un halogène, un alcoxy en $C_1$ à $C_6$ ou un alcoylthio en $C_1$ à $C_6$,
caractérisé en ce qu'on fait réagir un composé de formule générale:

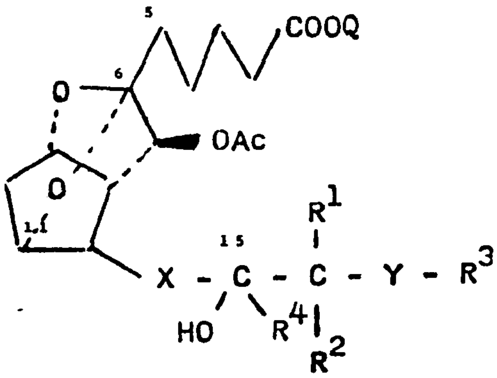

(III)

où $R^1$, $R^2$, $R^3$, $R^4$, X, Y et Q ont la signification donnée ci-dessus et Ac est un acétyle,
avec un alcanol inférieur en présence d'une quantité catalytique d'un catalyseur acide, en ce qu'on protège dans les 6-cétals obtenus de formule générale:

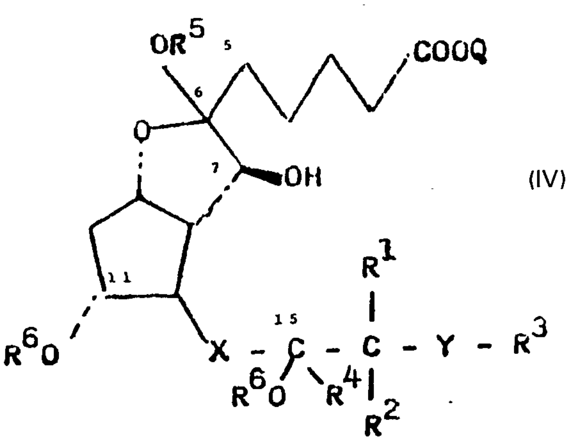

(IV)

où $R^1$, $R^2$, $R^3$, $R^4$, X, Y, Q et Ac ont la signification donnée ci-dessus, et $R^5$ est un alcoyle en $C_1$ à $C_6$, les groupes hydroxyle en 11 et 15 par réaction avec un trialcoylchlorosilane de formule générale:

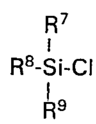

(VII)

un énoléther de formule générale:

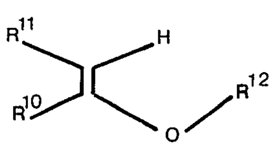

(VIII)

où $R^7$, $R^8$, $R^9$, qui peuvent être semblables ou différents, représentent un alcoyle en $C_1$ à $C_4$; $R^{10}$ et $R^{11}$, qui peuvent être semblables ou différents, représentent un hydrogène ou un méthyle et $R^{12}$ représente un méthyle ou un éthyle, ou avec le 3,4-dihydro-2H-pyrane
et en ce qu'on désacétyle les cétals obtenus de formule générale:

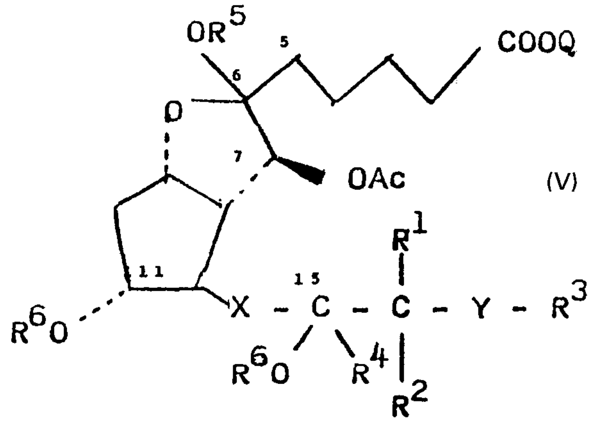

(V)

où $R^1$, $R^2$, $R^3$, $R^4$, $R^5$, X, Y, Q et Ac ont la signification donnée ci-dessus, et $R^6$ représente un groupe silyle de formule $R^7R^8R^9Si$-, un $\alpha$-alkoxyalcoyle de formule:

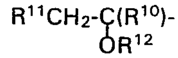

ou un groupe tétrahydropyran-2-yle,
en position 7 en milieu basique,
en ce qu'on oxyde le groupe hydroxyle libre formé en position 7 des cétals protégés obtenus de formule générale:

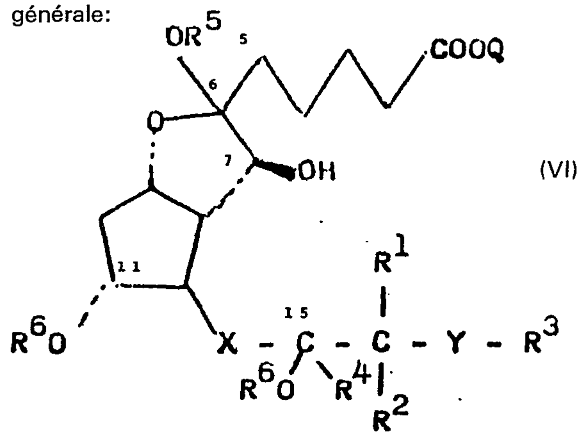

(VI)

où $R^1$, $R^2$, $R^3$, $R^4$, $R^5$, $R^6$, X, Y et Q ont la signification donnée ci-dessus,
éventuellement après retrait du groupe $R^5$-alcoyle par hydrolyse dans des solvants aprotiques, et en ce qu'on transforme les dérivés 7-oxo obtenus de formule générale:

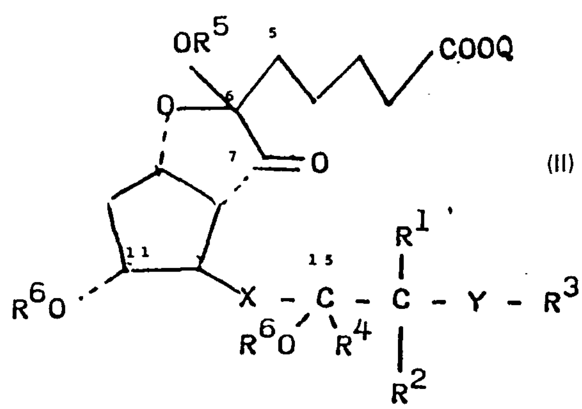

(II)

où $R^1$, $R^2$, $R^3$, $R^4$, $R^5$, $R^6$, X, Y et Q ont la signification donnée ci-dessus,
éventuellement après séparation des groupes protecteurs $R^6$, qui peut être suivie d'une acylation des groupes hydroxyle libres en 11 et en 15 formés lors de la séparation des groupes protecteurs $R^6$, lorsque $R^5$ est un alcoyle en $C_1$ à $C_6$, par séparation d'un alcool de formule $R^5$-OH, lorsque $R^5$ est remplacé par un hydrogène, par séparation d'eau,
en un composé de formule I, où si on le désire, on sépare les groupes protecteurs $R^{13}$ et/ou on saponifie les groupes ester et/ou on transforme l'acide libre en un sel ou un ester.

16. Préparations pharmaceutiques contenant comme substance active un ou plusieurs des dérivés de 7-oxo-$PGI_2$ des revendications 1 à 14.